# EUROPEAN PATENT APPLICATION

(11) **EP 4 154 902 A1**
(43) Date of publication of application: **29.03.2023**
(21) Application number: 22190446.9
(22) Date of filing: 25.10.2018
(51) Int. Cl.: A61K 38/17, A61P 35/00, A61K 45/00

(54) **WNT5A PEPTIDES IN REDUCTION OF CANCER STEM CELLS**

(30) Priority: 25.10.2017 EP 17198369
(62) Divisional of application: 18789182.5
(71) Applicant: Wntresearch AB, 222 25 Malmö (SE)
(72) Inventor: Sjölander, Anita, 216 11 Limhamn (SE)
(74) Representative: AWA Denmark A/S

(57) **Abstract**

A peptide derived from WNT5A protein according to SEQ ID NO: 1
for use in the reduction or elimination of tumour growth in a patient diagnosed with a cancer and having said tumour.

## Description

The present invention relates to a WNT5A-derived peptide for use in the prevention of recurrence or relapse of a cancer in a patient or for use in the reduction or elimination of cancer stem cells in a patient diagnosed with a cancer.

### Background

A primary tumour is rarely the cause of death of cancer patients. In the majority of cases, the mortality is the result of cancer relapse after different periods of recurrence-free survival following the surgical removal of the primary tumor. Current treatment of patients with breast cancer, colon cancer or prostate cancer includes surgery, chemotherapeutic drugs, endocrine treatment and some novel biological treatments. The latter treatment modalities are primarily targeting proliferative cancer cells and thus growth of the cancer. Although these treatments can have good effect on the primary tumour itself and on the majority of tumor cells remaining after surgery, a large proportion of the patients subsequently develop recurrent disease.

An essential reason for such cancer relapse is the existence of a subset of tumor cells having 'stem-like' characteristics. These tumour-initiating cells which are distinct from non-malignant stem cells, show low proliferative rates, high self-renewing capacity, multi- or pluripotency, ability to differentiate into actively proliferating tumour cells and are further characterized by their resistance to chemotherapy or radiation. These stem-like cells are also referred to as Cancer Stem Cells (CSCs). It is believed that elimination of CSCs could possibly eradicate the cancer disease and thus relapse of this disease. A fundamental problem in cancer research is therefore the identification and in particular targeting of CSCs responsible for recurrent disease. A tumor marker is a biomarker found in blood, urine, or body tissues that can be elevated by the presence of one or more types of cancer. There are many different tumor markers, each indicative of a particular cancer, and they are used for diagnosis the presence of cancer and to specify the specific type of cancer. Thus, an elevated level of a tumor marker may indicate a cancer.

Many different attempts have been made to overcome the problem of killing CSCs such as delivery of therapeutic agents *i.e.* small molecules, siR-NA or antibodies that affect embryonic signalling pathways implicated in self-renewal and differentiation into CSCs. There are, however, many drawbacks with the present attempts of eliminating CSCs. Firstly, the markers differ from one type of cancer to another and there is no universal marker that can be used for all cancers. Secondly, biologically distinct CSCs can exist within certain tumours as known from both acute myeloid leukemia, as well as some solid tumours. Finally, there is a risk of affecting normal stem cells when targeting drugs to CSCs because CSCs have the same expression profiles as normal stem cells.

On this background it is therefore an object of the present invention to provide a non-toxic and safe therapeutic agent to prevent or significantly delay relapse of the cancer disease due to survival of CSCs.

### Summary

In a first aspect of the invention the object of preventing or delaying relapse of cancer is achieved by a peptide derived from WNT5A protein according to SEQ ID NO: 1, which peptide has a length of 20 amino acids or less and comprises the amino acids sequence XDGXEL (SEQ ID NO: 2), or a formylated derivative thereof, wherein X is any amino acid, said peptide being for use in the prevention of recurrence or relapse of a cancer in a patient or for use in the reduction or elimination of cancer stem cells (CSCs) in a patient diagnosed with a cancer. In the context of the present invention recurrence or relapse has the same meaning. As it is believed that elimination of CSCs could possibly eradicate the cancer disease and thus relapse of this disease cf above. it is currently believed that the recurrent disease may be caused by the CSCs and it is thus contemplated that the reduction or eradication of cancer stem cells will delay or prevent the recurrence of cancer.

In one embodiment, the peptide according to the invention is for use in the prevention of recurrence or relapse of a cancer in a patient or for use in the reduction or elimination of CSCs in a patient diagnosed with a cancer, said cancer being selected from the list consisting of prostate cancer, breast cancer, colon cancer, ovarian cancer, thyroid cancer, liver cancer or haematological malignancies.

In a further embodiment, the expression of protein WNT5A in the cancer cells of the patient is 35% or less of the expression in surrounding non-cancer cells. The expression of the level of WNT5 in both cancer and non-cancer cells is measured by immunohistochemistry (IHC) using specific primary antibodies directed against WNT5 combined with a secondary antibody for its detection and quantification.

A low expression of WNT5A in breast, colon and prostate cancer tumours have been correlated with an increased number of diseases recurrences and a shortened survival time of the patient. However, this effect of WNT5A signalling cannot be attributed to its effect on cancer cell proliferation, that is often absent or limited in tumours with elevated β-catenin signalling (Dejmek et al., The Expression and Signaling Activity of Wnt-5a/DDR1 and Syk Play Distinct but Decisive Roles in Breast Cancer Patient Survival. Clinical Cancer Research. 11:520-528, 2005, Säfholm et al., The Wnt-5a-derived hexapeptide Foxy-5 inhibits breast cancer metastasis *in vivo* by targeting cell motility.

Clinical Cancer Research 14(20):6556-6563, 2008, Mehdawi et al., Non-canonical WNT5A signaling up-regulates the expression of the tumour suppressor 15-PGDH and induces differentiation of colon cancer cells, Molecular Oncology 10:1415-1429, 2016, Canesi et al., Treatment with the Wnt5a-mimicking peptide Foxy5 effectively reduces the metastatic spread of Wnt5a-low prostate cancer cells in an orthotopic mouse model. PLoS ONE doi.org/10.1371/journal.pone.084418, 2017)

WNT5A protein and a known WNT5A hexapeptide called Foxy-5, has in *in vitro* experiments been shown to decrease the expression of the PGE2 producing enzyme COX-2 and to up-regulate the PGE2 and lipoxin degrading enzyme 15-PGDH in colon cancer cells (Mehdawi et al., 2016). In this experimental *in vitro* study the authors also showed that both recombinant WNT5A and the Foxy-5 peptide induced a reduction in β-catenin signalling. Surprisingly, the inventors of the present invention have not been able to detect any reduction in the number of CSCs during such *in vitro* conditions.

The skilled person knows how to diagnose a cancer or a tumour, measure the tumour growth, differentiate or distinguish tumour tissue from normal tissue and how to measure the amount of WNT5A. The cancer may be diagnosed by Computed Tomography (CT) imaging. A low expression of WNT5A protein may be 35%, 30%, 20%, 15%, 10%, 5%, 2% or 1% of the WNT5A expression in surrounding non-cancer cells, or no expression of WNT5A.

Since a drug has to be produced in large scale, full length WNT5A protein has several disadvantages as drug candidate. Being a large protein WNT5A protein requires a complex and lengthy synthesis just in order to produce the correct primary amino acid sequence and post-translational modifications, and in addition WNT5A has a heparan sulphate-binding domain which may limit its distribution in the body. Therefore, a smaller peptide that mimics the effect of WNT5A is more preferred. WNT5A peptides comprising the amino acid sequence XDGXEL also comprises WNT5A mimicking effects. The peptides of varying lengths and comprising the sequence XDGXEL (SEQ ID NO: 2) may be produced synthetically, for example by liquid phase or solid phase peptide synthesis. The peptide of varying lengths has 20 amino acids or less and more preferred 10 amino acids or less. Most preferably, the peptide is a hexapeptide consisting of 6 amino acids. In one embodiment the amino acid sequence is XDGXEL, wherein the X in position 1 is M or norleucine and X in position 4 is C or A. Advantageously, the WNT5A peptide according to the invention is a peptide selected from the group consisting of:
MDGCEL (SEQ. ID. NO. 3),
GMDGCEL (SEQ. ID. NO. 4),
EGMDGCEL (SEQ. ID. NO. 5),
SEGMDGCEL (SEQ. ID. NO. 6),
TSEGMDGCEL (SEQ. ID. NO. 7),
KTSEGMDGCEL (SEQ. ID. NO. 8),
NKTSEGMDGCEL (SEQ. ID. NO. 9),
CNKTSEGMDGCEL (SEQ. ID. NO. 10),
LCNKTSEGMDGCEL (SEQ. ID. NO. 11),
RLCNKTSEGMDGCEL (SEQ. ID. NO. 12),
GRLCNKTSEGMDGCEL (SEQ. ID. NO. 13),
QGRLCNKTSEGMDGCEL (SEQ. ID. NO. 14),
TQGRLCNKTSEGMDGCEL (SEQ. ID. NO. 15),
GTQGRLCNKTSEGMDGCEL (SEQ. ID. NO. 16), and
LGTQGRLCNKTSEGMDGCEL (SEQ. ID. NO. 17),
or a formylated derivative thereof.

In a preferred embodiment, the WNT5A peptide is MDGCEL (SEQ. ID. NO. 3). In a more preferred embodiment methionine in position 1 is derivatized as formylated methionine (N-formyl methionine). This formylated hexapeptide is denoted Foxy-5. The modification/derivatization (formylation) of one amino acid improves the effect of the peptide and makes it more effective and resistant to degradation *in vivo.*

In another embodiment it is contemplated that the growth or niche of CSCs is favoured by active β-catenin, increased COX-2 and reduced 15-PGDH expression. Active β-catenin signalling as well as increased COX-2 and reduced 15-PGDH expression are most readily measured by IHC using primary and specific antibodies directed against active β-catenin, COX-2 or 15-PGDH combined with a matching secondary antibody for their detection and quantification.

In further embodiments the targeted cancer cells have β-catenin signalling levels or COX-2 expression that are increased by more than 35%, 40%, 50%, 65%, 70%, 90% or more than 100% compared to normal surrounding tissue and/or a 15-PGDH reduced expression that is reduced by more than 35%, 40%, 50%, 65%, 70%, 90% or up to 100% compared to normal surrounding tissue. The normal surrounding tissue is to be understood as the tissue immediately surrounding consisting of normal cells of the same type as the cancer cells.

In yet another embodiment it is contemplated that the peptide according to the invention is for use in the prevention of recurrence or relapse of a cancer in a patient or for use in the reduction or elimination of CSCs in a patient diagnosed with a cancer, which prevention, reduction or elimination comprises the following step: immediately after diagnosis of cancer and/or during surgery and/or after removing the tumour by surgery, administering an effective amount of the peptide, optionally wherein said step is repeated at least 3 times a week for 2 weeks or more. The period for treatment of the cancer stem cells with said peptide may be for 4, 6, 8, 12 weeks or up to 1, 2 or 3 months or more. Said peptide may also be administered during the treatment period with the tumour suppressing chemotherapeutic drug, since Foxy-5 do not impair the cytotoxic effect of chemotherapy. Tumour suppressing chemotherapeutic drugs are generally very toxic and in addition to the tumour cells also destroy healthy cells. Due to the toxicity of the tumour suppressing chemotherapeutic drug it may not be administered long enough or in a dosage high enough to destroy or eliminate all cancer cells or CSCs since the adverse effect would outweigh any positive effects.

The advantage of administering said peptide after treatment with a tumour suppressing drug is that the peptide is non-toxic to the healthy cells whilst it eliminates or reduces the number of CSCs that remain following treatment with the tumour suppressing chemotherapy, to which CSCs are resistant.

It is also contemplated that the administration of a tumour suppressing chemotherapeutic drug and the peptide according to the invention can be simultaneous or the peptide may be administered before, during and after surgical removal of the primary tumour until the chemotherapy is started or subsequent to the conclusion of the tumour suppressing chemotherapeutic treatment.

Thus such combinatorial treatment can result in an improved cancer treatment without increasing the side effect.

Administering in combination may be beneficial to the patient compliance as the time period where treatment is on-going may be shortened.

Administering sequential, i.e. the peptide before or following the tumour suppressing chemotherapeutic drug may be more efficient from a monetary perspective since the peptide is costly.

In an embodiment the invention provides a peptide derived from WNT5A protein according to SEQ ID NO: 1, which peptide has a length of 20 amino acids or less and comprises the amino acids sequence XDGXEL (SEQ ID NO: 2), or a formylated derivative thereof, wherein X is any amino acid, for use in the reduction or elimination of CSCs in a patient diagnosed with a cancer.

In further embodiments the invention provides the following numbered objects:
1. A peptide derived from WNT5A protein according to SEQ ID NO: 1, which peptide has a length of 20 amino acids or less and comprises the amino acids sequence XDGXEL (SEQ ID NO: 2), or a formylated derivative thereof, wherein X is any amino acid, said peptide being for use in the prevention of recurrence or relapse of a cancer in a patient or for use in the reduction or elimination of cancer stem cells in a patient diagnosed with a cancer.
2. The peptide according to object 1, for use in the prevention of recurrence or relapse of a cancer in a patient or for use in the reduction or elimination of cancer stem cells in a patient diagnosed with a cancer, said cancer selected from the list consisting of prostate cancer, breast cancer, colon cancer, ovarian cancer, thyroid cancer, liver cancer or haematological malignancies.
3. A peptide according to any one of objects 1 or 2 for use in the prevention of recurrence or relapse of a cancer in a patient or for use in the reduction or elimination of cancer stem cells in a patient diagnosed with a cancer, wherein expression of WNT5A in cancer cells is 35% or less of the expression in surrounding non-cancer cells.
4. The peptide according to any one of objects 1 to 3 for use in the prevention of recurrence or relapse of a cancer in a patient or for use in the reduction or elimination of cancer stem cells in a patient diagnosed with a cancer, wherein X in position 1 is M or norleucine and X in position 4 is C or A.
5. The peptide according to any one of objects 1 to 4 for use in the prevention of recurrence or relapse of a cancer in a patient or for use in the reduction or elimination of cancer stem cells in a patient diagnosed with a cancer, wherein said peptide is selected from the group consisting of:
   MDGCEL (SEQ. ID. NO. 3),
   GMDGCEL (SEQ. ID. NO. 4),
   EGMDGCEL (SEQ. ID. NO. 5),
   SEGMDGCEL (SEQ. ID. NO. 6),
   TSEGMDGCEL (SEQ. ID. NO. 7),
   KTSEGMDGCEL (SEQ. ID. NO. 8),
   NKTSEGMDGCEL (SEQ. ID. NO. 9),
   CNKTSEGMDGCEL (SEQ. ID. NO. 10),
   LCNKTSEGMDGCEL (SEQ. ID. NO. 11),
   RLCNKTSEGMDGCEL (SEQ. ID. NO. 12),
   GRLCNKTSEGMDGCEL (SEQ. ID. NO. 13),
   QGRLCNKTSEGMDGCEL (SEQ. ID. NO. 14),
   TQGRLCNKTSEGMDGCEL (SEQ. ID. NO. 15),
   GTQGRLCNKTSEGMDGCEL (SEQ. ID. NO. 16), and
   LGTQGRLCNKTSEGMDGCEL (SEQ. ID. NO. 17),
   or a formylated derivative thereof.
6. The peptide according to any one of objects 1 to 7 for use in the prevention of recurrence or relapse of a cancer in a patient or for use in the reduction or elimination of cancer stem cells in a patient diagnosed with a cancer, wherein said peptide is MDGCEL (SEQ.ID.NO.3), or a formylated derivative thereof.
9. The peptide according to any one of objects 1 to 8 for use in the prevention of recurrence or relapse of a cancer in a patient or for use in the reduction or elimination of cancer stem cells in a patient diagnosed with a cancer, wherein the cancer cells exhibit elevated β-catenin.
10. The peptide according to any one of objects 1 to 9 for use in the prevention of recurrence or relapse of a cancer in a patient or for use in the reduction or elimination of cancer stem cells in a patient diagnosed with a cancer, wherein the β-catenin signalling is elevated by 35% or more of the β-catenin signalling in surrounding non-cancer cells.
11. A combination of a peptide derived from WNT5A protein according to SEQ ID NO: 1, which peptide comprises the amino acids sequence XDGXEL (SEQ ID NO: 2), or a formylated derivative thereof, wherein X is any amino acid, wherein X is any amino acid in combination,
   with a tumour suppressing drug,
   for use in the prevention of recurrence or relapse of a cancer in a patient or for use in the reduction or elimination of cancer stem cells in a patient diagnosed with a cancer,
   wherein said peptide and said tumour suppressing drug are either combined or separate and/or are administered either simultaneously or sequentially.
12. The combination according to object 11 wherein said tumour suppressing drug is a combination of 5-fluorouracil (5-FU), leucovorin and oxaliplatin (FOLFOX), anthracycline such as epirubicin or doxorubicin, or taxane such as docetaxel or paclitaxel.
13. The combination according to object 11 wherein said tumour suppressing drug is a combination of 5-FU, leucovorin and oxaliplatin (FOLFOX) for use in treatment in a patient diagnosed with colon cancer.
14. The combination according to object 11 wherein said tumour suppressing drug treatment is combinations of anthracyclines such as epirubicin or doxorubicin, or taxanes such as docetaxel or paclitaxel for use in treatment in a patient diagnosed with breast cancer.

### Brief description of the figures

The figures described in the following are to support the detailed description. The invention will be described with reference to the figures in which:

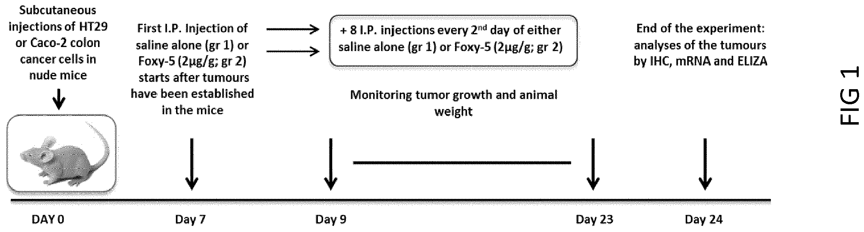

Figure 1 is a schematic overview of the animal experiments described in examples 1 to 3.

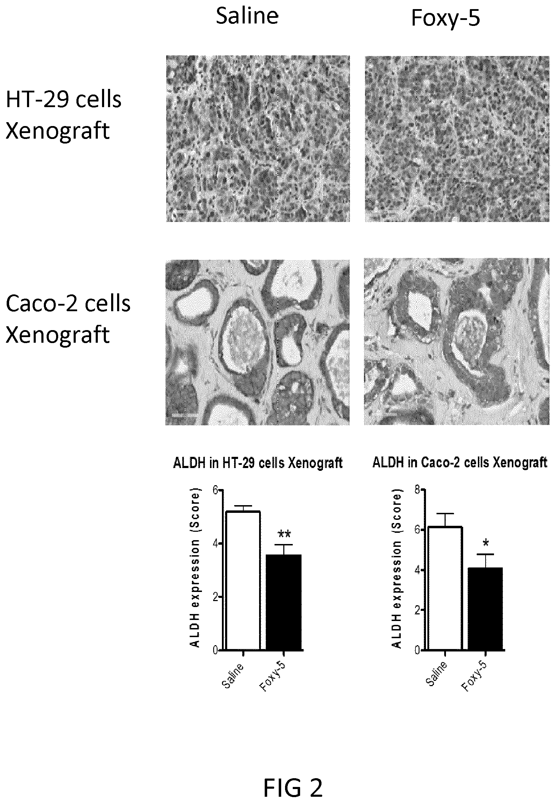

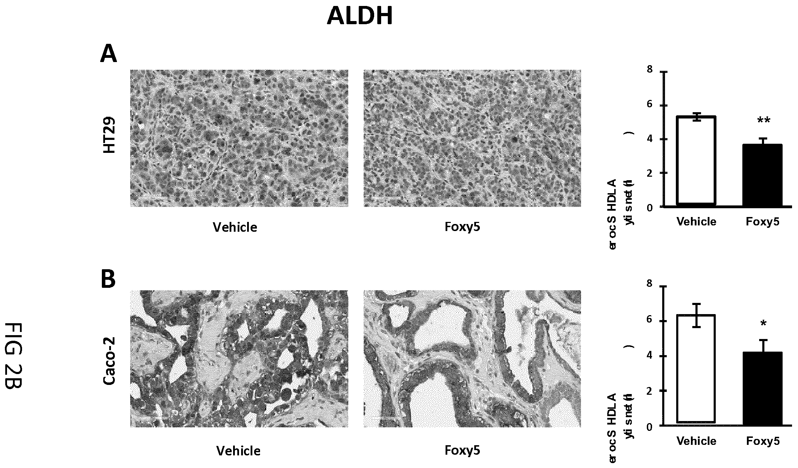

Figure 2 shows the in vivo effect of Foxy-5 treatment on ALDH protein expression in colon cancer tissue as visualized by immunohistochemistry (IHC). Representative photos from saline- and Foxy-5-treated animals are presented. From each such slide, 4 boxes were put on top of the stained tissue and for each of them the ALDH staining was scored as a percentage of stained cells multiplied by the staining intensity. The following score was used for percentage stained cells; 0 for positive stained cells <5%, 1 for positive cells 5-25%, 2 for positive cells 26-50%, 3 for positive cells 51-75%, 4 for positive cells >75%.
   For staining intensities, the stained areas were scored as; 1 for weak staining, 2 for medium staining and 3 for strong staining. For each box a final score was obtained by multiplying the score for percentage of positively stained cells with the score for staining intensity. Finally, a mean value was obtained from the 4 boxes for each tumour. The same scoring protocol was then used for all IHC analyses presented in the present study.

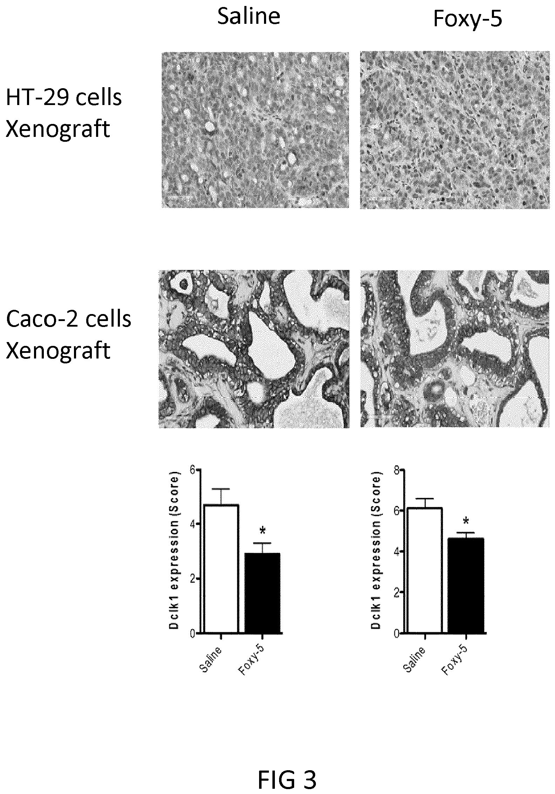

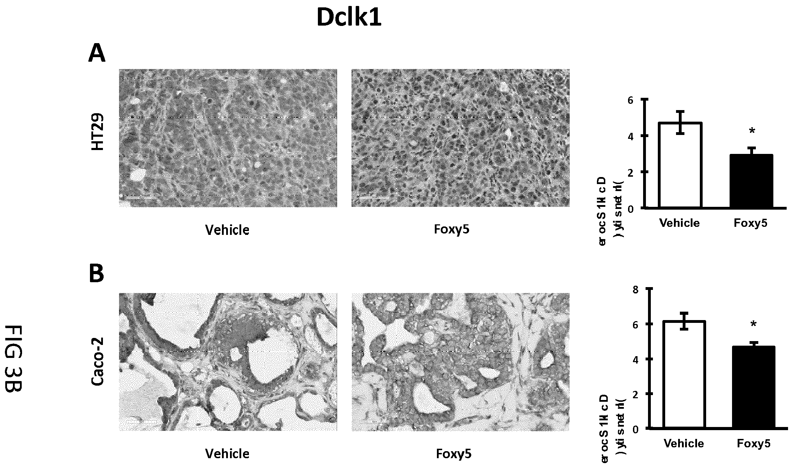

Figure 3 shows the *in vivo* effect of Foxy-5 treatment on Dck1 (also named DCAMKL1) protein expression in colon cancer tissue as visualized by IHC. Representative photos from saline- and Foxy-5-treated animals are presented. From each such slide 4 boxes were put on top of the stained tissue and for each of them the Dck1 (DCAMKL1) staining was scored as a percentage of stained cells multiplied by the staining intensity as described in detail for Figure 2.

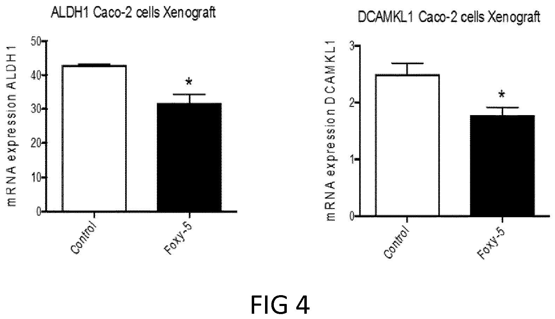

Figure 4 shows the *in vivo* effect of Foxy-5 treatment on ALDH and Dck1 (DCAMKL1) mRNA expression in colon cancer xenograph tissue compared with that of tumors from vehicle-treated control mice.

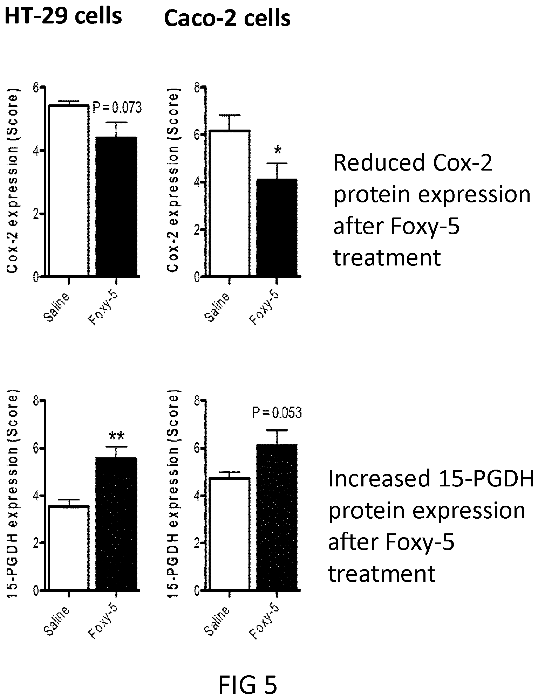

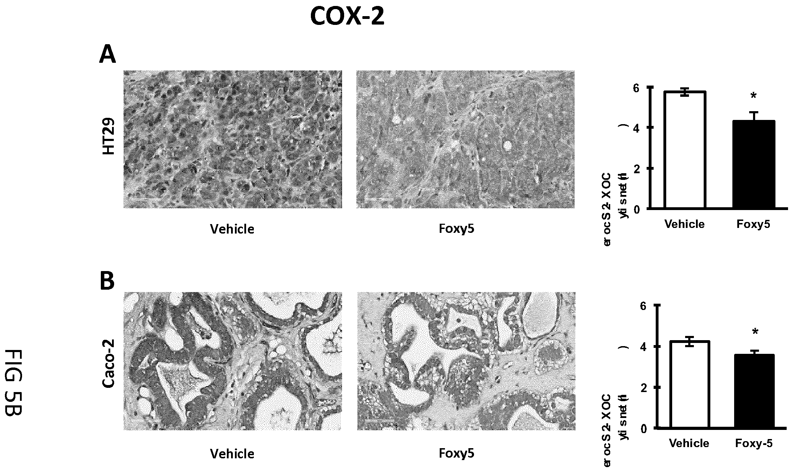

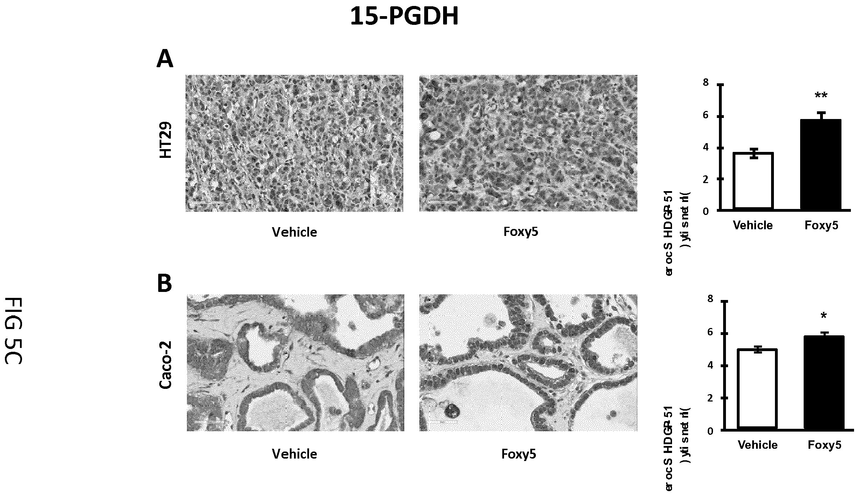

Figure 5 shows the *in vivo* effect of Foxy-5 treatment on Cox-2 and 15-PGDH protein expression in colon cancer tissue as visualized by IHC. The IHC stainings were scored exactly as described in detail for the IHC staining in Figure 1.

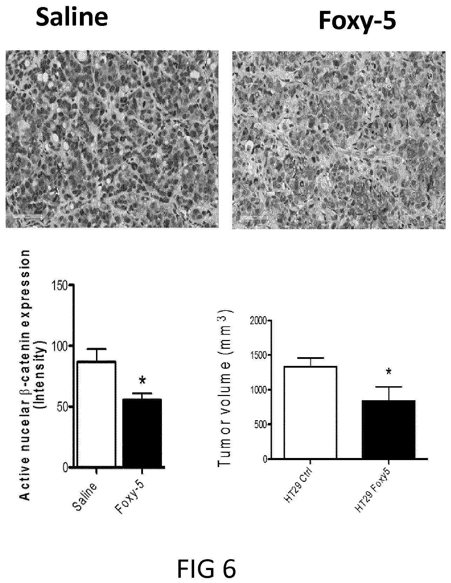

Figure 6 shows the *in vivo* effect of Foxy-5 treatment on active β-catenin nuclei expression in HT-29 colon cancer tissue as visualized by IHC. The IHC stainings were scored exactly as described in detail for the IHC staining in Figure 1. In the lower right panel, the size of the tumours for both the saline- and Foxy-5-treated animals are presented.

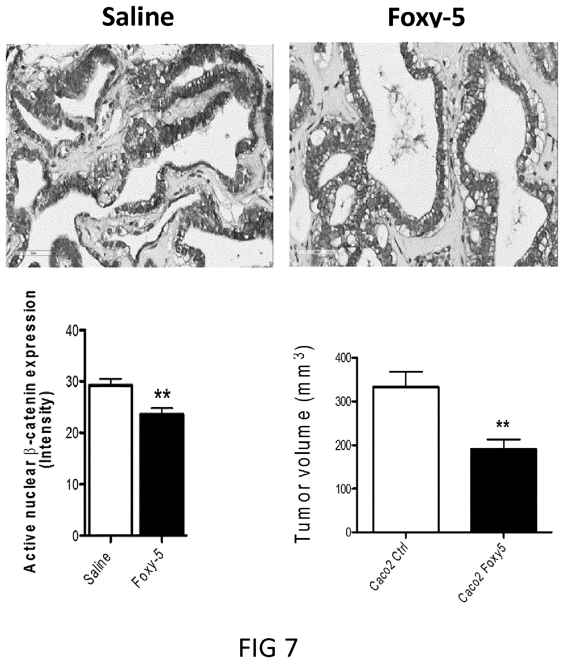

Figure 7 shows the *in vivo* effect of Foxy-5 treatment on active β-catenin nuclei expression in Caco-2 colon cancer tissue as visualized by IHC. The IHC staining were scored exactly as described in detail for the IHC staining in Figure 2. In the lower right panel, the size of the tumours for both the saline- and Foxy-5-treated animal tumour are presented.
Figure 6 and 7 further shows the *in vivo* effect of Foxy-5 treatment on tumour volume in HT-29 and Caco-2 colon cancer xenograph tissue, respectively. The knowledge that colon cancers have elevated β-catenin signalling makes it possible to validate the effects outlined in Figure 6 and 7, respectively, by determining the its relation to changes in tumour volume. The figures outlines the effects of Foxy-5 treatment on tumour volume of HT29 and Caco-2 derived tumours compared to that from control (vehicle-treated) mice.
Figure 2B is based on a repeated and extended analysis as in figure 2 and shows the *in vivo* effect of Foxy-5 treatment on ALDH protein expression in colon cancer xenograph tissue as visualized by immunohistochemistry (IHC). Representative images from vehicle (saline) and Foxy-5-treated animals are presented. From each such slide, 6 boxes were randomly put on top of the stained tissue and for each of them the ALDH staining was scored as a percentage of stained cells multiplied by the staining intensity. The following score was used for percentage stained cells; 0 for positive stained cells <5%, 1 for positive cells 5-25%, 2 for positive cells 26-50%, 3 for positive cells 51-75%, 4 for positive cells >75%.
For staining intensities, the stained areas were scored as; 1 for weak staining, 2 for medium staining and 3 for strong staining. For each box a final score was obtained by multiplying the score for percentage of positively stained cells with the score for staining intensity. Finally, a mean value was obtained from the 6 boxes for each tumour sample. The same scoring protocol was then used for all IHC analyses presented in the present study.
Figure 3B is based on a repeated and extended analysis as in figure 3 and shows the *in vivo* effect of Foxy-5 treatment on Dck1 (also named DCAMKL1) protein expression in colon cancer xenograph tissue as visualized by IHC. Representative images from vehicle- and Foxy-5-treated animals are presented. From each such slide 6 boxes were put on top of the stained tissue and for each of them the Dck1 staining was scored for staining intensity, as described in detail for Figure 2.
Figure 5B is based on a repeated and extended analysis as in figure 5 and shows the *in vivo* effect of Foxy-5 treatment on Cox-2 protein expression in colon cancer xenograph tissue as visualized by IHC. Representative images from vehicle- and Foxy-5-treated animals are presented. From each such slide 6 boxes were put on top of the stained tissue and for each of them the Cox-2 staining was scored as a percentage of stained cells multiplied by the staining intensity as described in detail for Figure 2.
Figure 5C is based on a repeated and extended analysis as figure 5 shows the *in vivo* effect of Foxy-5 treatment on 15-PGDH protein expression in colon cancer xenograph tissue as visualized by IHC. Representative images from vehicle- and Foxy-5-treated animals are presented. From each such slide 6 boxes were put on top of the stained tissue and for each of them the 15-PGDH staining was scored as a percentage of stained cells multiplied by the staining intensity as described in detail for Figure 2.

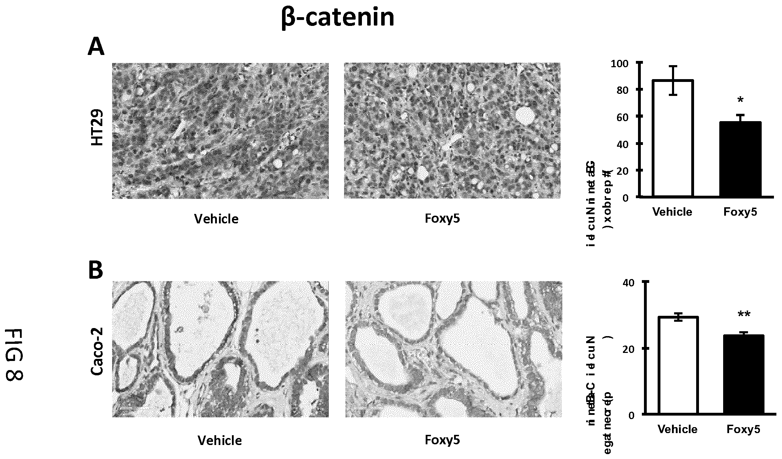

Figure 8 is based on a repeated and extended analysis as figure 6 and 7, respectively, and shows the *in vivo* effect of Foxy-5 treatment on active β-catenin nuclei expression in HT-29 and Caco-2 colon cancer xenograph tissue as visualized by IHC. Representative images from vehicle- and Foxy-5-treated animals are presented. From each such slide 6 boxes were put on top of the stained tissue and for each of them the active β-catenin nuclei staining was scored as a percentage of stained cells multiplied by the staining intensity as described in detail for Figure 2.

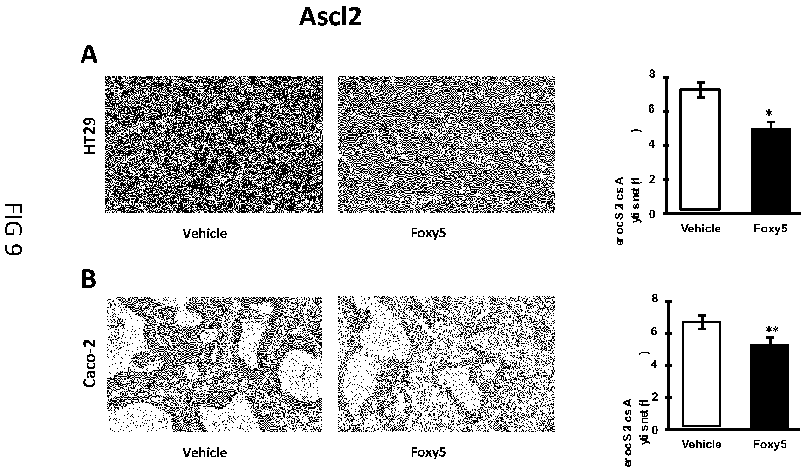

Figure 9 shows the *in vivo* effect of Foxy-5 treatment on Ascl2 protein expression in HT-29 and Caco-2 colon cancer xenograph tissue as visualized by IHC. The Ascl2 protein is a transcription factor activated by β-catenin signaling that has been show to promote the CSC niche. Representative images from vehicle- and Foxy-5-treated animals are presented. From each such slide 6 boxes were put on top of the stained tissue and for each of them the Ascl2 staining was scored as a percentage of stained cells multiplied by the staining intensity as described in detail for Figure 2.

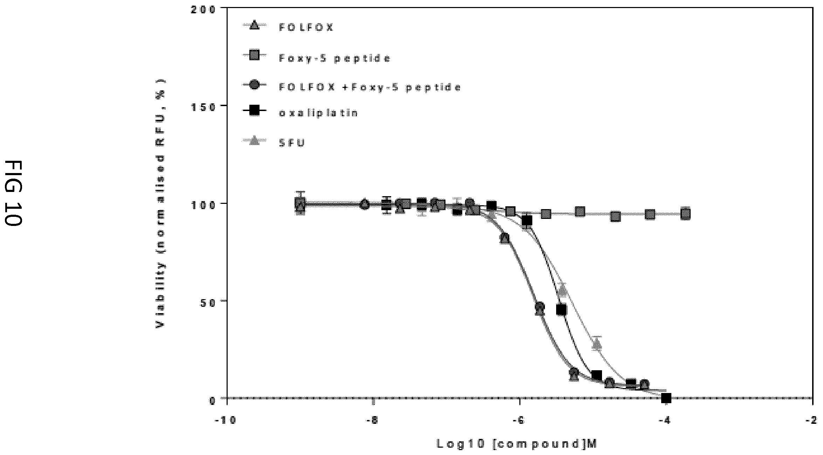

Figure 10 shows the absence of a Foxy-5 effect on FOLFOX-induced cytotoxicity. Colon cancer HT29 cells were treated with either FOLFOX alone (green triangles), 5-FU alone (orange triangles), oxaliplatin alone (black squares), Foxy-5 alone (red squares) or FOLFOX + Foxy-5 (blue circles). The cytotoxic effects of these different treatments were evaluated by CellTiter-Blue^{™} fluorescence cell viability assay. The dose-response curves of all these different treatments in HT29 are outlined in the figure. The data are normalized and shown as means ± SEM.

### Detailed description

The Wnt (Wingless-related integration site) protein family contains highly conserved proteins that play a role in embryonic development such as body axis patterning, cell proliferation and migration. The Wnt signalling pathways are either canonical or non-canonical and they primarily trigger the regulation of gene transcription and increased proliferation via canonical signalling or regulation of several non-proliferative functions via activation of different non-canonical signalling pathways in the cells. The Wnt proteins are further involved in tissue regeneration in adult bone marrow, skin and intestine. Genetic mutation in the Wnt signalling pathway may cause breast cancer, prostate cancer glioblastoma, type II diabetes and other diseases.

The canonical Wnt pathway activates β-catenin and is integral in regulating self-renewal of normal stem cells and the subversion of the canonical Wnt signalling has been implicated in tumourigenesis. In contrast, non-canonical Wnt signalling is characterized by an absence of an increase in β-catenin signalling and has been studied for its role in embryonic patterning, gastrulation, and organogenesis. Moreover, non-canonical Wnt is proposed to antagonize canonical signalling. WNT5A is an example of a non-canonical Wnt ligand. WNT5A is tumour-suppressive in acute myelogenous leukemia (AML), colon cancer, breast and prostate cancer, and ovarian carcinoma. Over-expression of WNT5A in a *WNT5A* homozygous mouse model was shown to correlate with a reduced number of breast CSCs in a study by Borcherding et a/., Paracrine WNT5A signalling inhibits expansion of tumour-initiating cells, Cancer Research 75:1972-1982, 2015 suggesting that heterozygous loss of WNT5A correlates with shorter survival of breast cancer patients. Interestingly, WNT5A has the opposite effect in malignant melanoma, gastric cancer as well as a few other cancer types, as exemplified by the fact that high expression of WNT5A in primary malignant melanoma is correlated with a shortened survival time.

WNT5A is a protein expressed by many normal cells in the body. WNT5A is secreted from the cells and exerts its action on the same or neighbouring cells by binding to and activating a receptor complex primarily involving a Frizzled receptor. The WNT5A protein is known to activate a receptor called Frizzled 5. Upon activation of the Frizzled 5 receptor a series of signalling events inside of the cells are activated, where one of the first events, is generation of short-lived increase in calcium inside of the cell, a so called calcium-signal. The calcium-signal in turn triggers a series of forthcoming signalling events leading to a change in the functions of the cells, such as adhesion and migration. Thus, activating such a Frizzled receptor leads to signalling events inside the cell, resulting in increased adherence of the cell to its neighbouring cells and its adhesion to the surrounding connective tissue resulting in decreased ability of the tumour cell to migrate to structures in the vicinity, such as lymph nodes and blood vessels. In healthy breast epithelial cells for example, WNT5A is highly expressed and secures a firm adherence between cells and to the surrounding basement membrane and thereby restricts migration of the cells.

In order to reconstitute WNT5A signalling in cancer tissue that lack an endogenous expression of WNT5A, a small peptide, i.e. equal to or less than 20 amino acids derived from the amino acid sequence of the WNT5A molecule has been developed and then additionally modified. An example of such a peptide is Foxy-5, which is a true WNT5A agonist in that it triggers the same signaling events and functional responses as WNT5A and in comparison, with WNT5A it is much simpler molecule and it can be administered systemically and still reach the tumor tissue. Thus, the term signalling properties, as used herein, means binding of the WNT5A or the Foxy-5 peptide to primarily a Frizzled receptor protein (Fz) followed by an intracellular signalling cascade in the cell eventually leading to reduction or elimination of CSCs.

The term surrounding non-cancer cells, as used herein, means morphologically normal cells, of the same type from which the tumour has originated, enclosing or encircling the tumour tissue.

### Examples

### Example 1

Tumours from two different human colon cancer cell types HT29 and Caco-2 cells were examined by immunohistochemistry (IHC) and mRNA (see figure 1).

On day 0: Subcutaneous injections of HT29 or Caco-2 colon cancer cells in nude mice were done.

On day 7 after tumours have been established in the mice, intraperitoneal (I.P) injections of vehicle (saline) alone (gr 1) or Foxy-5 (2µg/g; gr 2) were done.

On days 9-23 tumour growth and animal weight was monitored - then + 8 I.P. injections every 2nd day of either vehicle alone (gr 1) or Foxy-5 (2µg/g; gr 2).

On day 24 the two types of tumours (Caco-2 and HT29 derived) (divided in two parts of which one was fixed and the other frozen at -80°C) were analyzed by IHC for their protein expression of COX-2, 15PGDH, β-catenin, Ascl2, ALDH and Dckl1 and for their mRNA content of ALDH and Dckl1 (ALDH is a general stem cell marker and Dck1 is a specific marker for colon CSCs).

Foxy-5 was shown to significantly reduce the expression of the two stem cell biomarkers in both Caco-2- and HT29-derived colon cancers (see figures 2 to 4).

### Example 2

To further validate the findings outlined in figures 2-4 we analyzed possible mechanisms responsible for the decreased number of CSCs in the present experiments. The enzyme cyclooxygenase-2 (COX-2) is often upregulated in colon cancer. COX-2 activity leads to generation and release of prostaglandin E2 (PGE2). PGE2 promotes cancer progression and was shown to favor expansion of colon CSCs. (Wang et al., Prostaglandin E2 promotes colorectal cancer stem cell expansion and metastasis in mice. Gastroenterology 149:1884-1895, 2015). While perturbation of canonical WNTsignaling pathway is believed to account for the initiation of colorectal tumors, the increased expression of cyclooxygenase-2 (COX-2) that occurs in the majority of colorectal tumors is thought to play a crucial role during colorectal cancer development. Increased COX-2 expression leads to an increased abundance of its principal metabolic product, prostaglandin E2 (PGE2). Treatment of the animals in the present study with Foxy-5 resulted in a decrease not only in the expression of the PGE2 generating enzyme COX-2 (see figure 5 and 5B and but also in an increase in the PGE2 degrading enzyme 15-PGDH (see figure 5 and 5C). These data demonstrate that treatment with the Foxy-5 peptide can by a unique dual action cause a reduction in the intra-tumor level of PGE2 and thus explain the observed reduction of CSCs (figures 2-4 and 2B-3B).

### Example 3

*In vivo* effect of Foxy-5 treatment on β-catenin signalling in HT-29 or Caco-2 colon cancer tissue could be another possible explanation for the observed reduced number of CSCs. Decreased amounts of active β-catenin nuclei expression were observed in both Caco-2 and HT29 derived colon cancer tumors (see Figure 7 and 8) as a result of Foxy-5 treatment. The observations of Foxy-5-reduced β-catenin signalling were validated by reductions in tumour volumes (Figure 6 and 7). Essential for the present study is the fact that it was also observed that Foxy-5-induced reductions of the β-catenin downstream target Ascl2, a transcription factor promoting the cancer stem cell niche, indicate that the ability of Foxy-5 treatment to reduce the number of CSCs is also dependent on reduced β-catenin/Ascl2 signaling (see Figure 6-9). Consequently, the Foxy-5 peptide exhibit a unique property to reduce the number of CSCs by its ability to target three different elements (COX-2, 15-PGDH and β-catenin) that leads to reduction in two separate signaling pathways promoting CSCs.

### Example 4

The aim of this study was to test the possibility of simultaneous treatment of Foxy-5 and FOLFOX, by evaluating the *in vitro* effect of the Foxy-5 peptide on the cytotoxic effect of FOLFOX. FOLFOX is a made up from a combination of the two chemotherapeutic agents 5-FU and oxaliplatin and folinic acid, the latter is also called leucovorin not a chemotherapeutic agent but is used to potentiate the effect of 5-FU on human HT29 colon cancer cells. FOLFOX is the most commonly used chemotherapy in the treatment of colon cancer patients.

The initial experiment was to evaluate the cytotoxic effect of the Foxy-5 peptide, oxaliplatin, and 5-FU as monotherapy (Figure 10). The results confirmed that as folinic acid there was no cytotoxic effect of the Foxy-5 peptide on cell viability and consequently no IC₅₀ value could be determined. Whereas the IC₅₀ values of oxaliplatin and 5-FU were calculated from the dose-response curves to be 3.4 and 6.8 µM, respectively.

The combination results indicate that the Foxy-5 peptide do not enhance or reduce the inhibitory effect of FOLFOX on cell viability. The IC₅₀ value of FOLFOX treatment alone was 1.5 µM and a combined treatment with the Foxy-5 peptide and FOLFOX exhibited the same IC₅₀ value, as is also clear from their overlapping dose-response curves in Figure 10.

In conclusion, addition of Foxy-5 did not alter the effect of FOLFOX treatment on the viability of human HT29 colon cancer cells, thus no interaction can be anticipated. There is therefore no indication that Foxy-5 will diminish the cytostatic effect of FOLFOX treatment.

### Items

Item 1: A peptide derived from WNT5A protein according to SEQ ID NO: 1, which peptide has a length of 20 amino acids or less and comprises the amino acids sequence XDGXEL (SEQ ID NO: 2), or a formylated derivative thereof, wherein X is any amino acid, said peptide being for use in the prevention of recurrence or relapse of a cancer in a patient or for use in the reduction or elimination of cancer stem cells in a patient diagnosed with a cancer.

Item 2: The peptide according to item 1, for use in the prevention of recurrence or relapse of a cancer in a patient or for use in the reduction or elimination of cancer stem cells in a patient diagnosed with a cancer, said cancer selected from the list consisting of prostate cancer, breast cancer, colon cancer, ovarian cancer, thyroid cancer, liver cancer or haematological malignancies.

Item 3: A peptide according to any one of item 1 or 2 for use in the prevention of recurrence or relapse of a cancer in a patient or for use in the reduction or elimination of cancer stem cells in a patient diagnosed with a cancer, wherein expression of WNT5A in cancer cells is 35% or less of the expression in surrounding non-cancer cells.

Item 4: The peptide according to any one of item 1 to 3 for use in the prevention of recurrence or relapse of a cancer in a patient or for use in the reduction or elimination of cancer stem cells in a patient diagnosed with a cancer, wherein X in position 1 is M or norleucine and X in position 4 is C or A.

Item 5: The peptide according to any one of items 1 to 4 for use in the prevention of recurrence or relapse of a cancer in a patient or for use in the reduction or elimination of cancer stem cells in a patient diagnosed with a cancer, wherein said peptide is selected from the group consisting of:
MDGCEL (SEQ. ID. NO. 3),
GMDGCEL (SEQ. ID. NO. 4),
EGMDGCEL (SEQ. ID. NO. 5),
SEGMDGCEL (SEQ. ID. NO. 6),
TSEGMDGCEL (SEQ. ID. NO. 7),
KTSEGMDGCEL (SEQ. ID. NO. 8),
NKTSEGMDGCEL (SEQ. ID. NO. 9),
CNKTSEGMDGCEL (SEQ. ID. NO. 10),
LCNKTSEGMDGCEL (SEQ. ID. NO. 11),
RLCNKTSEGMDGCEL (SEQ. ID. NO. 12),
GRLCNKTSEGMDGCEL (SEQ. ID. NO. 13),
QGRLCNKTSEGMDGCEL (SEQ. ID. NO. 14),
TQGRLCNKTSEGMDGCEL (SEQ. ID. NO. 15),
GTQGRLCNKTSEGMDGCEL (SEQ. ID. NO. 16), and
LGTQGRLCNKTSEGMDGCEL (SEQ. ID. NO. 17),
or a formylated derivative thereof.

Item 6: The peptide according to any one of items 1 to 5 for use in the prevention of recurrence or relapse of a cancer in a patient or for use in the reduction or elimination of cancer stem cells in a patient diagnosed with a cancer, wherein said peptide is MDGCEL (SEQ ID NO: 3), or a formylated derivative thereof.

Item 7: The peptide according to any one of items 1 to 7 for use in the prevention of recurrence or relapse of a cancer in a patient or for use in the reduction or elimination of cancer stem cells in a patient diagnosed with a cancer, which prevention, reduction or elimination comprises the following step:
a) immediately after diagnosis of cancer and/or during surgery and or after removing the tumour by surgery, administering an effective amount of the peptide, optionally wherein step a) is repeated at least 3 times a week for 2 weeks or more.

Item 8: The peptide according to any one of items 1 to 7 for use in combination with at least one tumour suppressing chemotherapeutic drug for the prevention of recurrence or relapse of a cancer in a patient or for use in the reduction or elimination in a patient diagnosed with a cancer, which prevention, reduction or elimination comprises the following step:
a) administering an effective amount of a tumour suppressing chemotherapeutic drug;
b) prior to, simultaneously with and/or subsequently after treatment with said at least one tumour suppressing chemotherapeutic drug, administering an effective amount of the peptide, optionally wherein step b) is repeated at least 3 times a week for 2 weeks or more.

Item 9: A peptide derived from WNT5A protein according to SEQ ID NO: 1, which peptide has a length of 20 amino acids or less and comprises the amino acids sequence XDGXEL (SEQ ID NO: 2), or a formylated derivative thereof, wherein X is any amino acid, in combination with at least one tumour suppressing chemotherapeutic drug.

Item 10: A combination of a peptide derived from WNT5A protein according to SEQ ID NO: 1, which peptide has a length of 20 amino acids or less and comprises the amino acids sequence XDGXEL (SEQ ID NO: 2), or a formylated derivative thereof, wherein X is any amino acid, in combination with at least one tumour suppressing chemotherapeutic drug, for use in the prevention of recurrence or relapse of a cancer in a patient or for use in the reduction or elimination of cancer stem cells in a patient diagnosed with a cancer, wherein said peptide and said tumour suppressing chemotherapeutic drug are either combined or separate and/or are administered either simultaneously or sequentially.

Item 11: The combination according to item 10 wherein said at least one tumour suppressing chemotherapeutic drug is a combination of 5-fluorouracil (5-FU), leucovorin and oxaliplatin (FOLFOX) or anthracycline or taxane.

Item 12: The combination according to item 10 and 11 wherein the anthracycline is epirubicin or doxorubicin, or the taxane is docetaxel or paclitaxel.

Item 13: The combination according to item 10 wherein said tumour suppressing chemotherapeutic drug is a combination of 5-fluorouracil (5-FU), leucovorin and oxaliplatin (FOLFOX) for use in treatment of a patient diagnosed with colon cancer.

Item 14: The combination according to item 10 wherein said tumour suppressing chemotherapeutic drug is an anthracycline or a taxane for use in treatment of a patient diagnosed with breast cancer.

Item 15: The combination according to item 10 and 14 wherein the anthracycline is epirubicin or doxorubicin, or the taxane is docetaxel or paclitaxel.

## Claims

1. A peptide derived from WNT5A protein according to SEQ ID NO: 1 for use in the reduction or elimination of tumour growth in a patient diagnosed with a cancer and having said tumour, wherein the peptide has a length of 20 amino acids or less and comprises amino acid sequence XDGXEL (SEQ. ID. NO. 2), or a formylated derivative thereof, wherein X in position 1 is methionine (M) or norleucine and X in position 4 is cysteine (C) or alanine (A).

2. A peptide agonist derived from WNT5A protein according to SEQ ID NO: 1 for use in the reduction or elimination of tumour growth in a patient diagnosed with a cancer and having said tumour, wherein the peptide agonist has a length of 20 amino acids or less and comprises amino acid sequence XDGXEL (SEQ. ID. NO. 2), or a formylated derivative thereof, wherein X in position 1 is methionine (M) or norleucine and X in position 4 is cysteine (C) or alanine (A).

3. A peptide derived from WNT5A protein according to SEQ ID NO: 1 for use in the reduction or elimination of tumour growth in a patient diagnosed with a cancer and having said tumour according to claim 1 or 2, wherein the tumour is a primary tumour.

4. A peptide derived from WNT5A protein according to SEQ ID NO: 1 for use in the reduction or elimination of tumour growth in a patient diagnosed with a cancer and having said tumour according to any one of the preceding claims, wherein the reduction or elimination of said tumour is measured as a decreased tumour volume (mm³).

5. A peptide derived from WNT5A protein according to SEQ ID NO: 1 for use in the reduction or elimination of tumour growth in a patient diagnosed with a cancer and having said tumour according to any one of the preceding claims, wherein the cancer is selected from the list consisting of prostate cancer, breast cancer, colon cancer, ovarian cancer, thyroid cancer, liver cancer or haematological malignancies.

6. A peptide derived from WNT5A protein according to SEQ ID NO: 1 for use in the reduction or elimination of tumour growth in a patient diagnosed with a cancer and having said tumour according to claim 5, wherein the cancer is colon cancer.

7. A peptide derived from WNT5A protein according to SEQ ID NO: 1 for use in the reduction or elimination of tumour growth in a patient diagnosed with a cancer and having said tumour according to any one of the preceding claims, wherein the peptide is selected from the group consisting of:
MDGCEL (SEQ. ID. NO. 3),
GMDGCEL (SEQ. ID. NO. 4),
EGMDGCEL (SEQ. ID. NO. 5),
SEGMDGCEL (SEQ. ID. NO. 6),
TSEGMDGCEL (SEQ. ID. NO. 7),
KTSEGMDGCEL (SEQ. ID. NO. 8),
NKTSEGMDGCEL (SEQ. ID. NO. 9),
CNKTSEGMDGCEL (SEQ. ID. NO. 10),
LCNKTSEGMDGCEL (SEQ. ID. NO. 11),
RLCNKTSEGMDGCEL (SEQ. ID. NO. 12),
GRLCNKTSEGMDGCEL (SEQ. ID. NO. 13),
QGRLCNKTSEGMDGCEL (SEQ. ID. NO. 14),
TQGRLCNKTSEGMDGCEL (SEQ. ID. NO. 15),
GTQGRLCNKTSEGMDGCEL (SEQ. ID. NO. 16), and
LGTQGRLCNKTSEGMDGCEL (SEQ. ID. NO. 17), or a formylated derivative thereof.

8. A peptide derived from WNT5A protein according to SEQ ID NO: 1 for use in the reduction or elimination of tumour growth in a patient diagnosed with a cancer and having said tumour according to any one of the preceding claims, wherein the peptide is MDGCEL (SEQ. ID. NO. 3), or a formylated derivative thereof.

9. A peptide derived from WNT5A protein according to SEQ ID NO: 1 for use in the reduction or elimination of tumour growth in a patient diagnosed with a cancer and having said tumour according to any one of the preceding claims, wherein the reduction or elimination comprises the following step:
a) immediately after diagnosis of the cancer, administering an effective amount of the peptide, optionally wherein step a) is repeated at least 3 times a week for 2 weeks or more.

10. A peptide derived from WNT5A protein according to SEQ ID NO: 1 for use in the reduction or elimination of tumour growth in a patient diagnosed with a cancer and having said tumour according to any one of claims 1-8, wherein the peptide is given in combination with at least one tumour suppressing chemotherapeutic drug, and wherein the reduction or elimination comprises the following steps of:
a) administering an effective amount of the at least one tumour suppressing chemotherapeutic drug;
b) prior to, simultaneously with and/or subsequently after treatment with said at least one tumour suppressing chemotherapeutic drug, administering an effective amount of the peptide, optionally wherein step b) is repeated at least 3 times a week for 2 weeks or more.

11. A peptide derived from WNT5A protein according to SEQ ID NO: 1 in combination with at least one tumour suppressing chemotherapeutic drug for use in the reduction or elimination of a tumour in a patient diagnosed with a cancer and having said tumour, wherein the peptide has a length of 20 amino acids or less and comprises amino acid sequence XDGXEL (SEQ. ID. NO. 2), or a formylated derivative thereof, wherein X in position 1 is methionine (M) or norleucine and X in position 4 is cysteine (C) or alanine (A).

12. A peptide derived from WNT5A protein according to SEQ ID NO: 1 in combination with at least one tumour suppressing chemotherapeutic drug for use in the reduction or elimination of a tumour in a patient diagnosed with a cancer and having said tumour according to claim 11, wherein said peptide and said at least one tumour suppressing chemotherapeutic drug are either combined or separate and/or are administered either simultaneously or sequentially.

13. A peptide derived from WNT5A protein according to SEQ ID NO: 1 in combination with at least one tumour suppressing chemotherapeutic drug for use in the reduction or elimination of a tumour in a patient diagnosed with a cancer and having said tumour according to claim 11 or 12, wherein said at least one tumour suppressing chemotherapeutic drug is a combination of 5-fluorouracil (5-FU), leucovorin and oxaliplatin (FOLFOX) and the cancer is colon cancer.
